# EUROPÄISCHE PATENTSCHRIFT

(11) **EP 1 290 032 B1**
(45) Veröffentlichungstag und Bekanntmachung des Hinweises auf die Patenterteilung: **09.08.2006**
(21) Anmeldenummer: 01960241.6
(22) Anmeldetag: 23.05.2001
(51) Int. Cl.: C07K 16/30, C07K 14/705, G01N 33/574

(54) **ANTIKÖRPER GEGEN SPEZIELLE FORMEN DES PROPSA UND DEREN VERWENDUNG IN IMMUNOASSAYS**
ANTIBODIES AGAINST PARTICULAR FORMS OF PROPSA AND USE THEREOF IN IMMUNOASSAYS
ANTICORPS CONTRE DES FORMES SPECIALES DU PROPSA ET LEUR UTILISATION DANS DES DOSAGES IMMUNOLOGIQUES

(30) Priorität: 24.05.2000 DE 10025387; 05.07.2000 DE 10032040
(43) Veröffentlichungstag der Anmeldung: 12.03.2003
(73) Patentinhaber: Roche Diagnostics GmbH, 68305 Mannheim (DE); F.HOFFMANN-LA ROCHE AG, 4070 Basel (CH)
(72) Erfinder: HOESEL, Wolfgang, 82327 Tutzing (DE); PETER, Jochen Nat. Inst. of Environmental Health, Research Triangle Park, NC 27709 (US); VORM, Ole, DK-5230 Odense (DK); KROGH, Thomas, N., DK-5260 Odense S (DK)
(86) Internationale Anmeldenummer: PCT/EP2001/005943
(87) Internationale Veröffentlichungsnummer: WO 2001/090194

(56) Entgegenhaltungen:
- EP-A- 1 043 394
- WO-A-00/02052
- WO-A-00/49158
- WO-A-00/67030
- WO-A-98/49323
- LEINONEN J ET AL : "Reactivity of 77 antibodies to prostate-specific antigen with isoenzymes and complexes of prostate-specific antigen." TUMOR BIOLOGY, Bd. 20, Nr. suppl1, 1999, Seiten 28-34, XP002902209
- NURMIKKO P ET AL : "Production and characterization of novel anti-prostate-specific antigen (PSA) monoclonal antibodies that do not detect internally cleaved Lys145-Lys146 inactive PSA" CLINICAL CHEMISTRY, Bd. 46, Nr. 10, 2000, Seiten 1610-1618, XP002902210

### SEQUENZPROTOKOLL

<110> Roche Diagnostics GmbH
<120> Antikörper gegen spezielle Formen des proPSA und deren Verwendung in Immunoassays
<130> 523500DE
<140>
   <141>
<160> 9
<170> PatentIn Ver. 2.1
<210> 1
   <211> 7
   <212> PRT
   <213> Homo sapiens
<400> 1
   Ala Pro Leu Ile Leu Ser Arg
    1               5
<210> 2
   <211> 7
   <212> PRT
   <213> Homo sapiens
<400> 2
   Pro Leu Ile Leu Ser Arg Ile
    1               5
<210> 3
   <211> 6
   <212> PRT
   <213> Homo sapiens
<400> 3
   Pro Leu Ile Leu Ser Arg
    1               5
<210> 4
   <211> 5
   <212> PRT
   <213> Homo sapiens
<400> 4
   Leu Ile Leu Ser Arg
   1 5
<210> 5
   <211> 4
   <212> PRT
   <213> Homo sapiens
<400> 5
   Ile Leu Ser Arg
   1
<210> 6
   <211> 7
   <212> PRT
   <213> Homo sapiens
<400> 6
   Val Pro Leu Ile Gln Ser Arg
   1 5
<210> 7
   <211> 6
   <212> PRT
   <213> Homo sapiens
<400> 7
   Leu Ile Leu Ser Arg Ile
   1 5
<210> 8
   <211> 5
   <212> PRT
   <213> Homo sapiens
<400> 8
   Ile Leu Ser Arg Ile
   1 5
<210> 9
   <211> 4
   <212> PRT
   <213> Homo sapiens
<400> 9
   Leu Ser Arg Ile
   1

## Beschreibung

Die Erfindung betrifft Antikörper gegen spezielle Formen der enzymatisch nicht aktiven Vorstufen des prostataspezifischen Antigens (PSA), deren Herstellung, deren Verwendung in Immunoassays zur Bestimmung des [-5, -6, -7]-proPSA, sowie den Einsatz der Messwerte aus diesen Assays für eine verbesserte Diagnostik im Umfeld des Prostata-Karzinoms.

### Beschreibung:

Das Prostata-Karzinom (PCa) ist in den USA der am meisten diagnostizierte Krebs bei Männern, Parker, S.L. et al., CA Cancer J. Clin., 46:5-27, 1996. Das prostataspezifische Antigen, oder auch PSA genannt, wurde als verläßlicher Prognose-Marker im großen Umfang für die Behandlung von an Prostatakrebs erkrankten Patienten benutzt. Catalona, W.J. et al., N. Engl. J. Med., 324:1156-1161, 1991; Osterling, J.E., 1.Urol., 145:907-923, 1991. Als Tumormarker hat es den großen Vorteil, dass es im Blut von gesunden Männern nicht oder nur in ganz geringen Konzentrationen nachweisbar ist. Im fortgeschrittenen Krankheitsstadium werden in aller Regel stark erhöhte PSA-Werte gemessen.

Eine diagnostische Frage von enormer Bedeutung ist die frühzeitige, sichere Erkennung eines PCa und die Unterscheidung von PCa und gutartiger Prostata Hyperplasie (BPH). Es ist eines der größten Probleme der heutigen PSA-Tests, dass diese Unterscheidung zwischen BPH und PCa nicht gewährleistet ist, McCormack, R.T. et al., Urology, 45:729-744,1995. So kann bei PSA-Konzentration im Bereich von 2 - 15 ng / ml Serum nicht unbedingt auf das Vorliegen einer malignen Erkrankung geschlossen werden, da solche PSA Werte auch durch eine BPH verursacht sein können. Es sind daher zusätzlich zu der PSA-Bestimmung weitere, z.T. aufwendige und schmerzhafte Untersuchungen (z.B. rektale Biopsien) notwendig, um das Vorliegen eines PCa abzuklären.

Um die diagnostische Genauigkeit des PSA-Nachweises aus Serum zu verbessern, wurden verschiedene alternative Vorgehensweisen vorgeschlagen, wie z. B. PSA-Dichte, PSA-Geschwindigkeit, Verhältnis zwischen freiem und gesamtem PSA oder zwischen komplexiertem und gesamtem PSA, Benson, M.C. et al., J. Urol., 147:815-816, 1992; Carter, H.B. et al., J. Am. Med. Assoc., 267:2215-2220, 1992; Oesterling, J.E. et al., J. Am. Med. Assoc., 270:860-864, 1993.

PSA gehört zu einer als "Kallikreine" bekannten Gruppe von Proteinen/Enzymen. Die menschliche Kallikrein-Familie besteht aus drei Mitgliedern, beschrieben als hK1, hK2 und hK3 (PSA). Clements, J.A., Endocr. Rev., 10:393-419, 1989; Carbini, L.A. et al., J.Hypertens., 11:893-898, 1993.

hK1 wird hauptsächlich in den Nieren, der Bauchspeicheldrüse und den submandibularen Speicheldrüsen produziert. Fukushima, D. et al., Biochemistry, 24:8037-8043, 1985.

hK2, wie PSA, wird vorherrschend im Prostata Epithelium produziert (Morris, B.J., Clin. Exp. Pharm. Phys., 16:345-351, 1989) und hat 78 % Homologie mit PSA (Schedlich, L.J. et al., DNA, 6:429-437, 1987; Lilja, H., World J. Urol., 11:188-191, 1993). Die Eigenschaften des hK2 als potenzieller Prostatakrebs-Marker wurde im Rahmen eines Übersichtsartikels diskutiert, Young, C.Y.F. et al., The Prostata Supplement, 7: 17-24, 1996.

Das Prostata spezifische Antigen (PSA) oder hK3 ist ein Glykoprotein mit einem Molekulargewicht von ca. 29 kDa. Es wird in den Prostata-Epithelzellen gebildet und ist ein Bestandteil der Samenflüssigkeit. PSA hat die enzymatische Aktivität einer neutralen Serinprotease. Seine Hauptfunktion besteht in der Spaltung der Seminogeline I und II sowie des Fibronektins, die als wesentliche Komponenten des Ejakulats die Beweglichkeit der Spermien bloclcieren. Durch die Hydrolyse dieser Proteine bewirkt PSA die Verflüssigung des Samenkoagulums und ermöglicht so die Mobilität der Spermien.

PSA ist eine Chymotrypsin-ähnliche Protease, wogegen hK2 eine Trypsin-ähnliche Protease ist.

Es ist bekannt, dass das PSA in unterschiedlichen molekularen Formen im Serum vorkommt. Der bei weitem größte Anteil des PSA liegt in Form inalctiver Komplexe vor, wobei vor allem das α1-Anti-Chymotrypsin das PSA komplexiert und dadurch auch dessen enzymatische Aktivität inhibiert.

Weitere Komplexe können mit anderen Serin-Protease-Inhibitoren (Serpine), wie α1-Antitrypsin und Protein C-Inhibitor gebildet werden, die aber im Vergleich zu PSA-ACT nur in sehr untergeordnetem Maße im Serum vorhanden sind. Darüberhinaus bildet PSA auch noch mit einem anderen Typ von Protease-Inhibitor, nämlich dem α2-Makroglobulin (α2-M) einen Komplex, über dessen Gehalt im Serum in der Literatur unterschiedliche Angaben gemacht werden, vor allem da das PSA in diesem Komplex immunologisch nicht zugänglich ist. Unter der Bezeichnung Gesamt-PSA ist nachfolgend die Summe von freiem und mit Serpinen komplexiertem PSA gemeint, da der PSA-Komplex mit α2-M in allen bisherigen immunologischen Bestimmungen nicht erfaßt wird (Tewari und Bluestein, J. Clin. Ligand Assay 1995, Vol. 18, S. 186-196). Neben der Bezeichnung Gsamt-PSA wird oft auch der Begriff PSA total mit gleicher Bedeutung verwendet.

In Analogie hierzu gilt auch für die anderen beiden Kallikreine hK1 und hK2 die gleiche Definition für Gesamt-Kallikreine, welche oft ebenso als Kallikrein (total) oder PSA (total) bezeichnet wird.

Neben komplexiertem PSA kommt im Serum noch enzymatisch inaktives, freies PSA vor, das keine Komplexe eingehen kann. Petterson et al., Clin. Chem. Vol. 41, (No. 10), Seite 1480-1488, (1995), beschreiben, dass etwa 15 % bis 20 % des Gesamt-PSA in freier (nicht komplexierter) Form im Serum vorliegen. Es wurde deshalb von Petterson et al. und anderen Arbeitsgruppen diskutiert, dass dieses freie, enzymatisch nicht aktive PSA, eventuell eine pro-Enzymform darstellt oder vielleicht durch interne Spaltung der Lys-Lys-Verbindungen zwischen den Aminosäuren 145 und 146 des PSA, inaktiviert worden sein könnte.

Das PSA-Molekül wird als prä-pro-Form bestehend aus insgesamt 261 Aminosäuren synthetisiert. Das 17 Aminosäuren lange Signal-Peptid wird bereits im endoplasmatischen Retikulum abgespalten. Das enzymatisch nicht aktive proPSA (Zymogen) ist demnach noch 244 Aminosäuren lang.

Durch Abspaltung des proPeptids (7 Aminosäuren) entsteht das enzymatisch aktive "reife" PSA (z. B. Melegos + Diamandis, Clin. Biochem., 29 (3), 193-200, 1996).

Das proPSA-Molekül mit den 7 N-terminalen Aminosäuren des PSA-proPeptids wird auch als [-7] proPSA bezeichnet. proPSA-Moleküle mit N-terminal verkürzter Sequenz werden entsprechend der Länge des proPeptid-Anteils als [-6], [-5], [-4] etc. proPSA bezeichnet.

Die in menschlichem Serum vorkommenden PSA-Formen sind in ihrer genauen Struktur bisher im Detail nicht bestimmt oder analysiert worden. So ist vor allem nicht geklärt, wie die genaue Struktur des in Serum frei, d.h. also nicht in Komplexen vorliegenden PSA aussieht (Paus et al., (1998) J. of Urology 159, 1599-1605). Der Grund dafür ist vor allem, dass die Konzentration des freien PSA in Serum so niedrig ist, dass es sehr schwierig ist, genügend Material zu isolieren und es einer Analytik zugänglich zu machen. Noldus et al. J. of Urology 158, 1606-1609 (1997) hatten aus 230 ml eines Gemisches von hochtitrigen PSA Seren (PSA total > 2000 ng / ml) mit einer Ausbeute von ca. 25% freies PSA in unreiner Form isoliert und es durch N-terminale Sequenzierung analysiert. Sie fanden Hinweise auf das Vorliegen eines normalen N-Terminus sowie auf eine Spaltung der PSA-Kette zwischen den Aminosäuren 145 und 146, welche erklären könnte, dass dieses PSA in nicht komplexierter Form vorliegt. Hinweise auf proPSA-Formen, d. h. auf PSA-Moleküle mit zusätzlichen Aminosäuren am N-terminalen Ende wurden von ihnen nicht gefunden.

In WO 98/49323 wurden Antikörper gegen PSA eingesetzt, um die in biologischen Proben vorhandenen molekularen Formen des freien PSA zu identifizieren und molekular zu charakterisieren. Dort konnte gezeigt werden, dass ein signifikanter Anteil des freien PSA als [-4]-proPSA, d. h. als ein N-terminal um 4 Aminosäuren der proPSA-Sequenz verlängertes PSA-Molekül im Serum vorkommt.

Im Stand der Technik sind keine spezifischen diagnostischen Verfahren für freie PSA-Moleküle, die noch Anteile des proPeptids enthalten, gezeigt. Eben so wenig ist bekannt, ob außer dem in WO 98149323 beschriebenen [-4]proPSA noch andere proPSA-Formen in Probenflüssigkeiten humanen Ursprungs vorkommen.
Im Stand der Technik finden sich zudem keinerlei Daten zur diagnostischen Wertigkeit von freiem PSA mit unterschiedlich langen proPSA-Sequenzanteilen.

Aufgabe der Erfindung war es daher, diagnostische Werkzeuge herzustellen, die einen spezifischen Nachweis bestimmter proPSA-Formen ermöglichen und zu überprüfen, ob diese neuartigen Tests zu einer verbesserten Diagnostik im Umfeld des Prostata-Karzinoms, insbesondere bei der Unterscheidung von BPH und PCa beitragen können.

In einem ersten Schritt wurde deshalb untersucht, ob sich durch den Einsatz modernster analytischer Methoden Hinweise auf andere proPSA-Formen, welche nicht dem [-4]-proPSA aus WO 98/49323 entsprechen, finden lassen. Es konnte überraschenderweise Hinweise auf mindestens drei zusätzliche proPSA-Formen [-2, -5, -7]-proPSA gefunden werden. Aufgrund dieser Voruntersuchungen wurde die Frage der diagnostischen Relevanz dieser proPSA-Formen untersucht.

Um die erfindungsgemäße Aufgabenstellung zu bearbeiten, wurden Antikörper hergestellt und gezielt ausgewählt, welche mit [-5, -6, -7]-proPSA reagieren, aber kürzere proPSA-Formen [-4], [-3], usw. nicht signifikant erkennen.

Synthetische Peptide, welche den N-terminalen Sequenzen der verschieden langen proPSA-Formen entsprechen, wurden hergestellt und sowohl für Immunisierungs-, wie auch für Screening- und Charakterisierungszwecke eingesetzt.

Es wurden Antikörper entwickelt und isoliert, welche die erfmdungsrelevanten Spezifitäten aufweisen. Solche Antikörper reagieren mit [-5, -6, -7]-proPSA, zeigen aber keine signifikanten Bindeeigenschaften gegen Peptide des [-4]-proPSA oder kürzere proPSA-Peptide.

Eine bevorzugte Ausführungsform stellen deshalb Antikörper gegen proPSA dar, welche dadurch gekennzeichnet sind, dass sie an [-5, -6, -7]-proPSA binden, aber mit [-4]-proPSA oder weiter verkürzten pro-PSA-Formen, im Wesentlichen keine Reaktion zeigen.

Diese Antikörper wurden zudem nach üblichen Verfahren gereinigt, modifiziert, derivatisiert und in Immunoassays zum Nachweis von [-5, -6, -7]-proPSA eingesetzt.

Es wurde überraschenderweise festgestellt, dass native Proben einen messbaren Anteil an [-5, -6, -7]-proPSA enthalten. Darüber hinaus konnte weiterhin überraschend beobachtet und festgestellt werden, dass diese speziellen proPSA-Formen relativ stabil sind und sogar in solch signifikanter Konzentration in den untersuchten Proben vorkommen, dass diese über Immunoassays gemessen werden können.

Die über die erfindungsgemäßen Methoden ermittelten [-5, -6, -7]-proPSA-Werte wurden bezüglich der klinisch/diagnostischen Relevanz mit anderen aus dem Stand der Technik bekannten PSA-Assays verglichen.

Wie eingangs erwähnt, ist eines der Hauptprobleme bei der Verwendung von PSA-Messungen in der klinischen Diagnostik der Bereich mit niedrigen, schwach erhöhten PSA-Werten. Die Unterscheidung zwischen PBH und PCa ist mit Gesamt-PSA-Werten nicht möglich. Der erfindungsgemäße Test auf spezielle Formen des proPSA liefert in diesem klinisch besonders kritischen Entscheidungsbereich, d. h. bei Gesamt-PSA-Konzentration unter 20 ng/ml (z. B. bestimmt mit Elecsys®-Test auf Gesamt-PSA der Fa. Roche Diagnostics) wichtige und diagnostisch relevante Zusatzinformationen und trägt damit zu einer besseren Unterscheidung zwischen PCa und BPH bei.

Eine weitere bevorzugte Ausgestaltung der Erfindung ist deshalb die Verwendung der [-5, -6, -7]-proPSA-Werte für diagnostische Fragestellungen im Umfeld des Prostata-Korzinoms.

Mit den erfindungsgemäßen Antikörpern und den damit etablierten immunologischen Verfahren ist es erstmals möglich, Quotienten aus der Gesamtmenge an freiem PSA und [-5, -6, -7]-proPSA zu bilden. Da sich überraschend erwiesen hat, dass dieser Quotient die klinisch/diagnostische Aussage erheblich verbessert, stellt die Verwendung eines Quotienten aus freiem PSA zu [-5, -6, -7]-proPSA eine weitere besonders bevorzugte Ausgestaltung der Erfindung dar.

### Legende zu den Abbildungen:

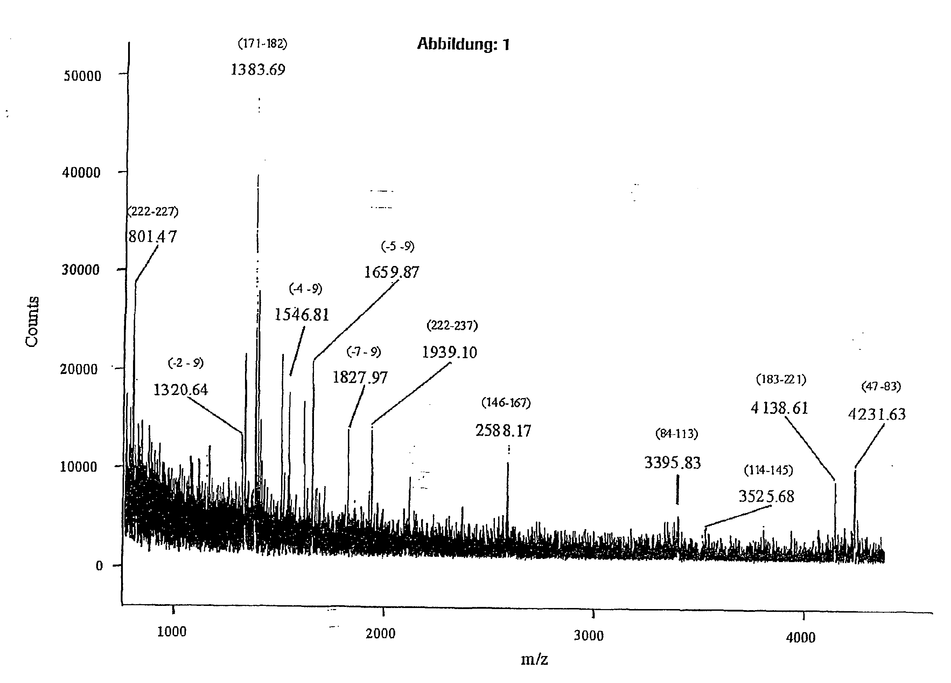

### Abbildung 1: (pro-) PSA-Peptide aus der MALDI-TOF-Analyse

Das freie PSA aus einem PCa Plasma wurde mit Hilfe von Immunsorption isoliert, auf einem SDS Gel aufgetrennt und die freie PSA Bande mit Endo Lys C verdaut. 0,5 µL dieser Probe wurden auf dem MALDI Probenteller mit 0,5 mL Matrix (10 mg/ml Sinapinsäure in 0,1% TFA:Acetonitril, 7:3,v:v) gemischt und das Massenspektrum ermittelt. Die Bezeichnungen der Peptide oberhalb der Massenzahlen (z.B. 114-145 geben den Bereich der Aminosäuresequenz von PSA an. Das dem [M+H]⁺ - Ionenpeak 1939.10 entsprechende Peptid ist das unvollständig gespaltene Peptid mit dem Aminosäurebereich 222 - 237 von PSA. Die pro Sequenz des PSA reicht von der Aminosäureposition -7 bis -1.

### Abbildung 2: Kompetitionstest mit freien proPSA-Peptiden

ProPSA aus einem PCa Plasma wurde über einen biotinylierten Antikörper gegen freies PSA an eine mit Streptavidin beschichteten Festphase gebunden. Die Bindung bzw. die Verdrängung des MAK 1.023.6 durch unterschiedlich lange proPSA-Peptide wurde ermittelt und ist in dieser Abbildung wiedergegeben.

### Abbildung 3: ROC-Analyse zur Aussagekraft verschiedener PSA-Parameter

Die Abbildung zeigt die über ROC-Analyse berechneten AUC-Werte (AUC= area under the curve). Je größer der AUC-Wert, desto besser ist ein Parameter (oder das Verhältnis von Parametern) bezüglich klinischer Genauigkeit und Aussagekraft im Hinblick auf Sensitivität und Spezifität.

### Im Folgenden ist die Erfindung im Detail beschrieben:

Der Vorläufer des PSA-Moleküls ist das sogenannte prä-pro-PSA, welches aus insgesamt 261 Aminosäuren besteht. Das Signal-Peptid ist 17 Aminosäuren lang und wird im endoplasmatischen Retikulum abgespalten, sobald das prä-pro-PSA in die sekretorischen Drüsen übertritt. Das dabei entstehende enzymatisch inaktive proPSA (diese inaktive Vorstufe des PSA wird auch als Zymogen bezeichnet)wird durch Freisetzung (Abspaltung) der sieben N-terminalen Aminosäuren des Pro-Peptids in das enzymatisch aktive, "reife" PSA überführt. Diese reife, enzymatisch aktive Form hat insgesamt 237 Aminosäuren und ein Molekulargewicht von ca. 29 kD.

Es ist bekannt, dass das PSA, aber auch die beiden anderen Kallikreine hK1 und hK2 in der Zirkulation zum größten Teil in Form inaktiver Komplexe, d. h. gebunden an verschiedene Protease-Inhibitoren, vorliegen. Der bei weitem größte Teil ist komplexiert mit α1-Anti-Chymotrypsin.

Das proPeptid des [-7]-proFSA ist 7 Aminosäuren - wie oben ausgeführt - lang. Es hat die Aminosäuresequenz Alanin-Prolin-Leucin-Isoleucin-Leucin-Serin-Arginin (APLILSR). Für hK2 lautet die proPeptid-Sequenz Valin-Prolin-Leucin-Isoleucin-Glutamin-Serin-Arginin (VPLIQSR). Die erste N-terminale Aminosäure des PSA ist ein Isoleucin. Es wurde überraschenderweise festgestellt, dass Antikörper mit dem erfindungsgemäßen Reaktionsspektrum, d.h. guter Bindung an die proPSA-Formen [-5], [-6] und [-7] erhalten werden können, wenn zur Immunisierung ein synthetisches Peptid, welches dem [6]-proPSA entspricht, eingesetzt wird. Das konkret verwendete Peptid enthielt zudem noch die erste Aminosäure aus dem reifen PSA. Seine Sequenz lautet somit PLILSRI.

Bekanntermaßen sind kurze Peptide per se wenig oder nicht immunogen. Zum Zwecke der Immunisierung ist es deshalb meist erforderlich, dass Peptide an geeignete Trägerstrukturen, z. B. Rinderserumalbumin oder keyhole limpet hemocyanine gekoppelt werden. Gegebenenfalls wird die Immunantwort zusätzlich durch Zugabe sogenannter adjuvanter Reagenzien verstärkt. Eine bevorzugte Ausgestaltung der Erfindung sind deshalb Immunogene, die [-5, -6, -7] proPeptide des PSA enthalten. Besonders bevorzugt sind Immunogene, die durch Verwendung des Peptids der SEQ ID No: 2 erhältlich sind.

Durch geeignete Kombination von anderen Immunogenen, wie z.B. einem [-7]-proPSA-Peptid oder von Mimetopen, z. B. des [-6]- proPSA mit den untenstehenden Screening-Kriterien können die erfindungsgemäßen Antikörper ebenfalls erhalten werden.

Ein Gegenstand der Erfindung sind somit Antikörper mit Spezifität für [-5, -6, -7]-proPSA, die mit [-4]-proPSA und kürzeren proPSA-Formen im Wesentlichen nicht reaktiv sind. Die unterschiedlichen proPSA-Formen [-7], [-6] und [-5]proPSA werden von solchen Antikörpern effizient gebunden, während das [-4]proPSA im Wesentlichen nicht reaktiv ist.

Das PLILSRI-Peptid wurde nach Standardmethoden synthetisch hergestellt (Peptide, Hans-Dieter Jakubke, Spektrum Verlag, Heidelberg, 1996). Erfahrungsgemäß sind kurze Peptide als solche wenig immunogen. Zur Immunisierung wird deshalb das synthetische PLILSRI-Peptid an ein geeignetes Trägerprotein, wie z. B. Keyhole Limpet Hemocyanine, Rinderserum Albumin oder Edestin gebunden.

Die Immunisierung erfolgt in den hierfür üblicherweise verwendeten Laboratoriumstieren, vorzugsweise werden Mäuse, Ratten, Kaninchen oder Schafe immunisiert. Zur Steigerung der Immunantwort können die üblicherweise verwendeten adjuvanten Substanzen (z. B. Freunds Adjuvants) eingesetzt werden.

Zur Herstellung monoklonaler Antikörper werden Milzzellen, vorzugsweise aus Mäusen, der entsprechend immunisierten Tiere, nach Verfahren, die dem Fachmann geläufig sind, immortalisiert. Besonders bevorzugte Verfahren sind hierbei die Hybridomtechnik (Köhler und Milstein, Nature 256 (1975), 495 - 497) oder die Transformation mit Eppstein-Barr-Virus (EBV-Transformation [Monoclonal Antibody Production Techiques and Application, Lawrence B. Schook, ed., Marcel Deldcer Verlag, 1987]).

Die Qualität der Immunantwort schwankt stark von Tier zu Tier, sowie von Spezies zu Spezies. Aus diesem Grund ist es notwendig, geeignete Screeningverfahren einzusetzen, welche sicherstellen, dass die gewonnenen Antikörper auch die erforderlichen Eigenschaften aufweisen.

Ein weiterer Gegenstand der Erfindung ist deshalb ein Herstellungsverfahren für Antikörper, welches ein geeignetes Screeningsystem umfasst. Dieses Screeningsystem stellt sicher, dass die durch obige Immunisierung gewonnenen Antikörper geeignete Bindeeigenschaften an [-5, -6, -7]-proPSA zeigen und mit [-4]-proPSA und kürzeren proPSA-Formen im Wesentlichen nicht reaktiv sind.

Ein besonders geeignetes Screeningsystem enthält das 7 Aminosäure lange PLILSRI-Peptid, welches besonders bevorzugt in biotinylierter Form an Streptavidin-Festphasen beschichtet ist. Alternativ kann das PSA-pro-Peptid auch an Proteine gebunden und über diese auf Festphasen adsorbiert werden. In all diesen Screeningsystemen werden besonders bevorzugt solche Varianten eingesetzt, welche sicherstellen, dass sowohl das Trägerprotein, wie auch die Kopplungschemie sich von der zur Immunogenherstellung verwendeten Einsatzstoffen unterscheiden, d. h. besonders bevorzugt werden alternative Trägerproteine und andersartige Linkerstrukturen eingesetzt.

Ein weiteres besonders geeignetes Screeningsystem ist ein kompetitiver Immunoassay mit biotinyliertem PSA- [-6 bis +1]pro-Peptid [PLILSRI-Bi] als Antigen. In diesem Testsystem wird das biotinylierte Peptid über Streptavidin an die Festphase beschichtet. Es wird dann geprüft, inwieweit andere/verkürzte Peptide aus dem pro-Peptid-Bereich des PSA in diesem Test die erfindungsgemäßen Antikörper verdrängen können. Es werden Antikörper ausgewählt, welche mit [-5, -6, -7]-proPSA reagieren, [-4]-proPSA aber im Wesentlichen nicht erkennen.

Ein alternatives Screening-System, welches in abgewandelter Form (siehe weiter unten) ebenfalls zum Nachweis von proPSA eingesetzt werden kann, ist ein sogenannter Sandwich-Test In diesem Test werden PSA (z. B. aus Spermien) sowie proPSA und PSA aus PCa-Plasma als Antigene ausgetestet.

Die Anbindung des (pro)-PSA an die Streptavidin-beschichtete Festphase erfolgt über die F(ab)-Fragmente, eines Antikörpers gegen freies PSA. Nachweisseitig werden die zu testenden neuen Antikörper gegen proPSA eingesetzt. In einem ersten Screening werden Antikörper ausgewählt, welche mit dem proPSA aus dem PCa-Serum reaktiv sind und mit dem PSA aus Sperma nicht reagieren.

Das obige System kann weiterhin eingesetzt werden, um die erfindungsgemäßen Antikörper über geeignete Verdrängungsrealctionen herauszuselektieren. Zu diesem Zweck wird proPSA aus PCa-Serum wie oben an die Festphase gebunden. Die weitere Spezifitätsprüfung erfolgt dann wie weiter oben für Tests mit biotinyliertem proPSA-Peptid als Antigen schon beschrieben.

Ein weiterer Gegenstand der Erfindung sind daher Antikörper, welche im obigen Kompetitionstest eine gute Kompetition mit dem [-7]-PSA-pro-Peptid, sowie mit dem [-6]-PSA-pro-Peptid und mit dem [-5]-PSA-pro-Peptid aber im wesentlichen keine Kompetition mit dem [-4]-PSA-pro-Peptid zeigen. Unter Kompetition wird hierbei verstanden, dass die [-5, -6, -7]-proPSA-Peptide, effizient mit dem untersuchten Antikörper um die Bindung an das PLILSRI-Peptid auf der Festphase konkurrieren. Im Wesentlichen nicht reaktive Antikörper gegen [-4]-proPSA und kürzere proPSA-Sequenzen sind solche Antikörper, die im Kompetitionstest weniger als 10 % der Verdrängungswirkung, besonders bevorzugt weniger als 5 % und ganz besonders bevorzugt weniger als 3 % der Verdrängungswirkung verglichen zum einem [-5]-proPSA-Peptid zeigen.

Unter dem Begriff "Antikörper" sind im Sinne der Erfindung neben den intakten Immunoglobulinen auch sämtliche Antikörperfragmente zu verstehen. Hierzu gehören beispielsweise Fab, Fab' oder F(ab)'₂₋Fragmente. Der Begriff Antikörper ohne Zusatz "monoklonal" oder "polyklonal" umfaßt immer beide Arten von Antikörpern, sowie eventuelle chimäre IConstrukte und alle oben angeführten Fragmente.

Unter Antikörpern, die im Wesentlichen gleiche Bindeeigenschaften zeigen, werden solche Antikörper verstanden, die mit dem am 16. Mai 2000 unter ACC 2456 bei der Deutschen Sammlung für Mikroorganismus und Zellkulturen (DSMZ), Braunschweig, hinterlegten Klon um die Bindung an [-5, -6, -7]-proPSA immunologisch konkurrieren.

Eine besonders bevorzugte Ausgestaltung der Erfindung sind Antilcörper, welche im Wesentlichen die gleichen Bindeeigenschaften aufweisen, wie der unter ACC 2456 bei der DSMZ hinterlegte Klon.

Die erfindungsgemäßen Antikörper können eingesetzt werden, um [-5, -6, -7]-proPSA spezifisch nachzuweisen. Besonders bevorzugt sind hierbei immunologische Verfahren, welche auf dem Kompetitionsprinzip oder dem sogenannten "Sandwich-Prinzip" beruhen. Die vielfältigen Ausgestaltungsmöglichkeiten für Immunoassays sind dem Fachmann bekannt, so dass hier auf eine detaillierte Schilderung verzichtet werden kann.

Ein weiteren Gegenstand der Erfindung sind daher immunologische Testverfahren, welche zur Quantifizierung/zum Nachweis des [-5, -6, -7]-proPSA aus einer Probe geeignet sind.

Als Proben können erfindungsgemäß alle dem Fachmann geläufigen biologischen Flüssigkeiten verwendet werden. Bevorzugt werden als Probe Körperflüssigkeiten wie Vollblut, Blutserum, Blutplasma oder Urin eingesetzt.

Besonders bevorzugt wird das [-5, -6, -7]-proPSA in einem Sandwich-Test nachgewiesen. Als besonders vorteilhaft hat sich dabei erwiesen, den spezifischen Antikörper gegen [-5, -6, -7]-proPSA als spezifischen Fangantikörper einzusetzen. Unter spezifisch wird hierbei verstanden, dass der Fangantikörper die im Screeningteil beschriebenen Spezifitätsanforderungen erfüllt.

Ein weiterer Gegenstand der Erfindung ist daher die Verwendung von Antikörpern gegen [-5, -6, -7]-proPSA in Immunoassays zum Nachweis dieser Moleküle.

Es hat sich ferner als besonders vorteilhaft erwiesen, beim Nachweis des [-5, -6, -7]-proPSA nachweisseitig einen Antikörper einzusetzen, welcher seinerseits spezifisch mit dem sogenannten freien PSA reagiert. Unter einem Antikörper mit Spezifität für freies PSA ist dabei zu verstehen, dass ein solcher Antikörper PSA-Moleküle, welche mit dem α1-Anti-Chymotrypsin (ACT) komplexiert sind, nicht erkennt.

Der Spiegel an [-5, -6, -7]-proPSA erwies sich als gut meßbar. Mit Hilfe des obigen Testsystems wurden deshalb kritische Humanseren, d. h. Humanseren mit ca. 4 bis ca. 10 ng/ml Gesamt-PSA untersucht. Eine Analyse der Meßwerte ergab, dass [-5, -6, -7]-proPSA-Werte wertvolle Zusatzinformationen im klinisch relevanten und kritischen Entscheidungsbereich liefern. Die [-5, -6, -7]-proPSA-Werte können herangezogen werden, um das Vorhandensein eines Prostata-Karzinoms zu diagnostizieren bzw. auszuschließen.

Wie eingangs schon erwähnt, ist ein deutlich erhöhter PSA-Wert von >20ng/ml von sehr hoher klinischer/diagnostischer Aussagekraft. Ein solcher Wert ist ein vergleichsweise sicheres diagnostisches Indiz für das Vorliegen eines PCa. Für Werte unter 20 ng/ml läßt die Aussagekraft stark nach. Ein Wert unter 10 ng/ml kann ebensogut durch BPH, wie durch ein PCa bedingt sein. Gerade in diesem Bereich wäre es jedoch besonders wichtig, eine gutartige Prostatahyperplasie von einem PCa im Frühstadium zu unterscheiden.

Einschlußkriterium für die ausgewählten Seren waren ein PSA (total)-Wert von 4 -10 ng/ml, welcher mit den entsprechenden Elecsys®-Test der Firma Roche Diagnostics ermittelt worden war. Neben PSA (total) wurde freies PSA (ebenfalls mit dem entsprechenden Elecsys®-Test von Roche Diagnostics) bestimmt.

Im klinisch besonders relevanten Bereich (4 -10 ng/ml) wurde die Aussagekraft der getesteten PSA-Marker einzeln und im Verhältnis zueinander über sogenannte ROC-Analysen untersucht.

Unter einer ROC-Analyse (ROC = receiver operator curve) versteht man die Berechnung und Erstellung eines sogenannten Sensitivitäts-Spezifizitäts-Diagramms. Ein Test mit einer guten diagnostischen Trennschärfe zeichnet sich durch einen möglichst großen AUC-Wert (AUC = area under the curve) aus. Je höher dieser Wert, desto besser die Leistungsmerlcmale des bewerteten Tests bezüglich seiner Spezifität und Sensitivität.

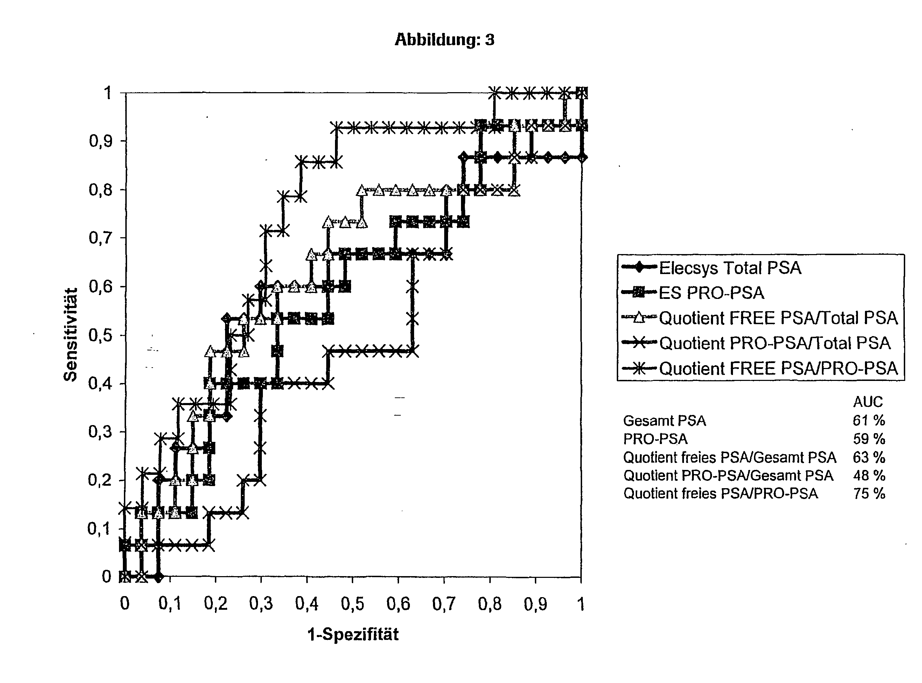

Das Verhältnis von [-5, -6, -7]-proPSA zu freiem PSA konnte aufgrund des neu entwickelten Immunoassays erstmals gebildet und ausgewertet werden. Erstaunlicherweise hat sich dieses Verhältnis in den durchgeführten ROC-Analysen als besonders aussagekräftig erwiesen, d. h. mit dem Verhältnis von [-5, -6, -7]-proPSA zu freiem PSA ist eine bessere Differenzierung zwischen PHB und PCa möglich, als mit den aus dem Stand der Technik bekannten Parametern. Dieser Vorteil wird bei Betrachtung von Abbildung 3 offenkundig.

Eine weitere bevorzugte Ausgestaltung der Erfmdung ist daher die vertiefende Analyse von kritischen PSA-Seren, d. h. von Seren mit einem Gesamt-PSA unter ca. 20 ng/ml, besonders bevorzugt mit einer PSA von 2 -15 ng/ml und insbesondere von 4 -10 ng/ml durch Auswertung des Quotienten von [-5, -6, -7]-proPSA zu freiem PSA oder des inversen Quotienten.

Die erfindungsgemäßen Antikörper können zudem eingesetzt werden, um die drei verschiedenen proPSA-Formen [-5]-proPSA, [6]-proPSA und/oder [-7]-proPSA einzeln zu bestimmen. Zu diesem Zweck wird ein erfindungsgemäßer Antikörper eingesetzt, um die [-5], [-6] und [-7]-proPSA-Moleküle aus der Probe anzureichern und diese einer weiteren Analyse zugänglich zu machen. Besonders bevorzugt erfolgt die Anreicherung über Immunaffinitätschromatographie oder selektive Immunsorption und der Einzelnachweis der obigen drei proPSA-Formen über massenspektrometrische (MS) Verfahren.

Während das erfindungsgemäße immunologische Verfahren die drei proPSA Formen [-5, -6, -7] als Summe erfaßt, ist es über die MS möglich, die drei proPSA Formen einzeln zu quantifizieren.

Ein weiterer Gegenstand der Erfindung ist daher die separate Quantifizierung von [-5], [-6] und [-7]-proPSA und die Verwendung dieser Einzelwerte zur Diagnose oder zum Ausschluß eines Prostata Karzinoms.

Die erfindungsgemäßen Antikörper reagieren ebenfalls mit dem [-6]-prohK2, mit der Aminosäuresequenz Prolin-Leucin-Isoleucin-Glutamin-Serin-Arginin (PLIQSR). In Analogie zu den oben beschriebenen Nachweisverfahren und Analyse kann mit den erfindungsgemäßen Antikörpern auch prohK2 bestimmt und zur Diagnose herangezogen werden.

Die Menge an den drei proPSA-Formen [-5], [-6] und [-7] kann zudem mit den zugrundeliegenden Erkrankungen und Verläufen verglichen werden, um damit ein aggressiver Krankheitsverlauf von einem eher langsamen Krankheitsverlauf zu unterscheiden.

Die folgenden Beispiele erläutern die Erfindung weiter:

### Beispiel 1

### Einzelnachweis unterschiedlicher proPSA-Formen über Massenspektroskopie

### a) Isolierung von freiem PSA aus einem PCa-Serum durch Immunsorption

Zu 28.7 mg magnetischen Streptavidinbeads wurden in einem 10 ml Röhrchen 4 ml einer Lösung des Antikörpers MAK<PSA>-M-30-IgG-Biotin (c = 25µg/ml) in PBS pH 7 +1%RSA + 0.1%Tween20 gegeben und für eine Stunde inkubiert. Die Beads wurden anschließend durch einen Magneten präzipitiert, der Überstand abpipettiert und die Beads dreimal mit jeweils 3 ml Waschlösung (PBS pH 7 + 20 mM Octylglucosid) gewaschen. Nach Zugabe von 8 ml Plasma (Gehalt an freiem PSA: 180 ng/ml) wurde wieder für 60 min bei Raumtemperatur inkubiert. Das Plasma wurde dann abpipettiert und die Beads 4 x mit je 1 ml Waschlösung gewaschen. Anschließend wurden die Beads mit 500 µl Propionsäure 1 M versetzt und die Suspension wieder für eine Stunde geschüttelt. Der Überstand wurde nach Präzipitation der Beads abgenommen, lyophilisiert und dann in 10 µl Aqua dest. aufgenommen. PSA aus Spermien wurde von der Fa. Scripps Laboratories, San Diego, bezogen.

### b) Identifizierung von freien proPSA Formen durch Matrix assisted Laser Desorption -Time of Flight Massenspektrometrie (MALDI-TOF MS).

Ein erster Hinweis, dass das immunsorptiv gereinigte, freie PSA aus dem Plasma sich in seiner Größe von dem aus Sperma unterscheidet zeigte sich in der MALDI-TOF MS. Dort wurde festgestellt, dass das freie PSA aus dem PCa Plasma mit 29100 Da ein um ca. 600 da höheres Molekulargewicht zeigte als freies PSA aus Sperma (28500 Da).

Nach Auftrennung des freien PSA aus PCa-Plasma und Sperma durch SDS-PAGE, reduktiver Alkylierung der Banden und Verdau mit Endoprotease Lys-C ergaben sich die in Abb. 1 gezeigten Peptidmuster. Zur Verdeutlichung sind die aus den jeweiligen PSA Formen nachgewiesenen Peptide in Tabelle 1 zusammengefaßt.

**Tabelle 1: (proPSA-) Peptide aus der MALDI-TOF-Analyse**

| [M+H] ⁺-Ionen der Peptide von freiem PSA aus Plasma; Werte in Dalton | [M+H]⁺-Ionen der Peptide von PSA-Referenz (Sperma); Werte in Dalton | Theoretische Werte der [M+H]⁺-Ionen-massen Werte in Dalton | AS-Sequenz (von - bis) |
|---|---|---|---|
| | | | |
| 801.47 | 801.50 | 801.96 | 222 - 227 |
| | 1077.54 | 1077.22 | 1- 9 |
| 1321.64 | | 1320.49 | (-2) - 9 |
| 1383.68 | 1384.35 | 1383.65 | 171-182 |
| 1546.80 | | 1546.81 | (-4) - 9 |
| 1659.87 | | 1659.97 | (-5) - 9 |
| 1827.97 | | 1828.16 | (-7) - 9 |
| 1940.10 | | 1940.29 | 222- 237 |
| 2588.23 | 2589.50 | 2587.98 | 146-167 |
| 3395.83 | 3397.83 | 3397.83 | 84-113 |
| 3525.68 | 3527.00 | 3525.95 | 114-145 |
| 4138.61 | 4141.71 | 4137.59 | 183-221 |
| 4231.63 | 4236.75 | 4234.86 | 47-83 |

Wie aus Abb. 1 sowie Tabelle 1 zu erkennen ist, enthält freies PSA aus PCa-Plasma, vier Peptide, die in PSA aus Sperma nicht vorhanden sind. Es handelt sich dabei um Peptide, die um 2, 4, 5 oder 7 Aminosäuren am N-Terminus des PSA verlängert sind und somit proPSA Formen darstellen.

### Beispiel 2

### Herstellung von monoklonalen Antikörpern (MAK) gegen proPSA

### a) Immunisierung von Mäusen

12 Wochen alte, weibliche Balb/C Mäuse wurden mit 100 µg des Peptids P-L-I-L-S-R-I-C (im weiteren als proPSA Peptid bezeichnet), welches an dem Cystein über einen Spacer an KLH (keyhole limpet hemocyanine) gekoppelt war, zusammen mit dem Adjuvans CFA (Komplettes Freund'sches Adjuvans) intraperitoneal erststimuliert. Nach 6 Wochen und danach in monatlichem Abstand, folgten drei weitere Immunisierungen intraperitoneal. Dabei wurde jeder Maus 100 µg des obigen Immunogens zusammen mit IFA (Inkomplettes Freund'sches Adjuvans) verabreicht. Anschließend erfolgten die letzten Immunisierungen intravenös mit je 100 µg des Immunogens in PBS-Puffer am 3. und 2. und letzten Tag vor der Fusion.

### b) Fusion und Klonierung

Die Fusion von Milzzellen der gemäß a) immunisierten Mäuse mit Myelomzellen erfolgt in Anlehnung an Galfre, Methods in Enzymology 73, 1981, 3. Dabei werden ca. 1 x 10⁸ Milzzellen der immunisierten Maus mit 2 x 10⁷ Myelomzellen (P3X63-Ag8-653, ATCC CRL1580) gemischt und abzentrifugiert (10 min bei 300 g und 4°C). Die Zellen werden dann einmal mit RPMI-1640-Medium ohne fötalem Kälberserum (FKS) gewaschen und bei 400 g in einem 50 ml Spitzröhrchen erneut abzentrifugiert, 1 ml PEG (Polyethylenglykol) (Molekulargewicht 4000, Merck, Darmstadt) dazugegeben, und durch Pipettieren gemischt. Nach 1 min im Wasserbad bei 37°C werden 5 ml RPMI 1640 ohne FKS zugetropft, durchmischt, auf 50 ml mit Medium (RPMI 1640 + 10% FKS) aufgefüllt und anschließend zentrifugiert. Die sedimentierten Zellen werden in RPMI 1640 Medium mit 10% FKS aufgenommen und in Hypoxanthin-Azaserin-Selektions-Medium (100 mmol/l Hypoxanthin, 1 µg/ml Azaserin in RPMI 1640 + 10% FICS) eingesät. Als Wachstumsfaktor wird dem Medium Interleukin 6 (100 U/ml) zugegeben. Nach ca. 10 Tagen wurden die Primärkulturen auf spezifische Antikörpersynthese getestet.

### c) Screening-Test auf anti-proPSA Peptid -Antikörper

Für das Screening der Mausseren, der Primärkulturen sowie der klonierten MAKs wurden mit rekombinantem Streptavidin beschichtete MTP (Fa. MicroCoat, Penzberg, Best.-Nr. 12-K 96 N, Charge MC 289) mit 1 µg/ml biotinyliertem proPSA Peptid in PBS plus 0,5 % Crotein C beschichtet (100 µl pro well, 10 min Inkubation bei Raumtemperatur unter Schütteln) und anschließend 3 x mit 0,9 % NaCl / 0,05% Tween 20 gewaschen. Das proPSA Peptid (PLILSRIC-Bi) war in diesem Konjugat mit einem anderen Spacer mit dem Biotin verbunden, als er bei dem oben beschriebenen Immunogen zur Immunisierung der Mäuse verwendet worden war, um Spacer spezifische Antikörper zu vermeiden. Danach wurden 100 µl der zu untersuchenden Antikörper-Lösung in ein beschichtetes well gegeben und 1 Stunde bei Raumtemperatur unter Schütteln inkubiert. Nach 3-maligem Waschen mit 0,9 % Natriumchlorid / 0,05% Tween 20 wurde zum Nachweis von gebundenem Antikörper aus der Probe jeweils 100 µl eines POD-markierten Fab-Fragments eines polyklonalen Antikörpers aus dem Schaf gegen Maus-Fcy (Roche Diagnostics GmbH, Ident.-Nr. 1431323, entsprechend 25 mU /ml) zugegeben, 1 Stunde bei Raumtemperatur unter Schütteln inkubiert und anschließend 3 x mit 0,9% Natriumchlorid / 0,05% Tween® 20 gewaschen.

Schließlich wurden jeweils 100 µl / well ABTS® (Roche Diagnostics GmbH, Kat.-Nr. 1204521 und 1204530) zugegeben und nach 30 min bei Raumtemperatur die Extinktion bei 405/492 nm in einem MR700 Microplate Reader der Firma Dynatech gemessen.

### d) Test der Maus-Seren und Kulturüberstände auf Erkennung von proPSA in PCa-Seren

Für das Screening der Mausseren, der Primärkulturen sowie der klonierten MAKs auf Reaktion mit proPSA-Formen in PCa-Serum wurden mit rekombinantem Streptavidin beschichtete MTP (Fa. MicroCoat, Penzberg, Best.-Nr. 12-K 96 N, Charge MC 289) mit 1 µg/ml biotinyliertem Fab Fragment des monklonalen Antilcörpers M-30, der nur freies PSA erkennt, in PBS plus 0,5 % Crotein C beschichtet (100 µl pro well, 10 min Inkubation bei Raumtemperatur unter Schütteln) und anschließend 3 x mit 0,9 % NaCl /0,05% Tween 20 gewaschen. Danach wird mit 100 µl des unverdünnten oder 1:10 mit PBS verdünnten PCa-Serums für 1 h unter Schütteln bei Raumtemperatur inkubiert. Im nächsten Schritt wurden 100 µl der zu untersuchenden Antikörper-Lösung in ein beschichtetes well gegeben und 1 Stunde bei Raumtemperatur unter Schütteln inkubiert. Nach 3-maligem Waschen mit 0,9 % Natriumchlorid / 0,05% Tween 20 wurde zum Nachweis von gebundenem Antikörper aus der Probe jeweils 100 µl eines POD-markierten Fab-Fragments eines polyklonalen Antikörpers aus dem Schaf gegen Maus-Fcy (Roche Diagnostics GmbH, Ident.-Nr. 1431323, ensprechend 25 mU / ml) zugegeben, 1 Stunde bei Raumtemperatur unter Schütteln inkubiert und anschließend 3 x mit 0,9% Natriumchlorid / 0,05% Tween® 20 gewaschen.

Schließlich wurden jeweils 100 µl/well ABTS® (Roche Diagnostics GmbH, Kat.-Nr. 1204521 und 1204530) zugegeben und nach 20 min bei Raumtemperatur die Extinktion bei 405/492 nm in einem MR700 Microplate Reader der Firma Dynatech gemessen. Als Kontrolle wurde PSA-freies Frauenserum eingesetzt.

Zur Überprüfung, dass die hergestellten Antikörper keine Realction mit freiem PSA aus Sperma zeigten, wurde als Antigen anstatt des PCa-Serums, freies PSA der Fa. Scripps, San Diego, Kat. Nr. P 0714, Charge 98 43 649, 1 µg / ml, gelöst in PBS plus 0,5% Crotein C, eingesetzt. Die Ergebnisse sind in Tabelle 2 zusammengefaßt.

**Tabelle 2: Reaktion eines hochtitrigen PSA-haltigen PCa-Serums mit Seren von Mäusen, die mit proPSA- Peptid-Immunogen immunisiert worden waren, im Vergleich zu einem Nullserum.**

| Ergebnisse [mE] | | | |
|---|---|---|---|
| Maus# | Prostata-CA-Serum | PSA-freies Serum | PSA |
| 493-1 | 286 | 39 | 38 |
| 493-2 | 231 | 36 | 38 |
| 493-3 | 71 | 10 | 106 |
| 493-4 | 35 | 35 | 35 |
| 493-5 | 106 | 39 | 40 |
| 493-6 | 166 | 37 | 37 |
| 493-7 | 143 | 37 | 37 |
| 493-8 | 209 | 37 | 37 |
| 493-9 | 74 | 35 | 38 |
| Nullserum | 37 | 38 | 56 |

Das Ergebnis zeigt zum einen, dass die meisten der untersuchten Seren aus gemäß la immunisierten Mäusen spezifisch mit dem PSA-haltigen PCa-Serum reagieren. Da sie zum anderen nicht mit freiem PSA aus Sperma, welches einen normalen N-Terminus des reifen PSA besitzt, reagieren, belegen diese Resultate zudem, dass in diesem PCa-Serum proPSA-Formen vorliegen müssen.

Primärkulturen wurden in analoger Weise getestet und Kulturen, die mit proPSA Peptid sowie dem proPSA-haltigem Serum eine positive Reaktion zeigten aber mit (Sperma-) PSA keine Reaktion aufwiesen, wurden mittels fluoreszenzaktiviertem Zellsorter in 96er-Zellkulturplatten kloniert. Hierbei wurde dem Medium als Wachstumszusatz Interleukin-6 (100 U/l) zugegeben. Auf diese Weise wurden die Klone der Tabelle 3 erhalten:

**Tabelle 3: Klone mit Reaktivität gegen pro-PSA**

| Klon | IgG-Subklasse | Extinktion/Test 1c) |
|---|---|---|
| 1.001.7 | IgG1 | 1.60 |
| 2.003.6 | IgG1 | 1.59 |
| 2.005.11 | IgG1 | 2.49 |
| 1.023.6 | IgG1 | 2.09 |
| 2.002.4 | IgG1 | 1.42 |

### e) Immunglobulingewinnung aus Ascites von Mäusen

Die erhaltenen Hybridomzellen wurden in einem Standardverfahren zur Produktion von IgG durch Ascitesbildung bei Mäusen eingesetzt. Die Reinigung dieser Antikörper aus dem Ascites erfolgte nach proteinchemisch üblichen Methoden (z.B. gemäß Methods in Enzymology 121 (1986), 587 - 695).

### Beispiel 3

### Test auf Spezifität für [-5, -6, -7]proPSA

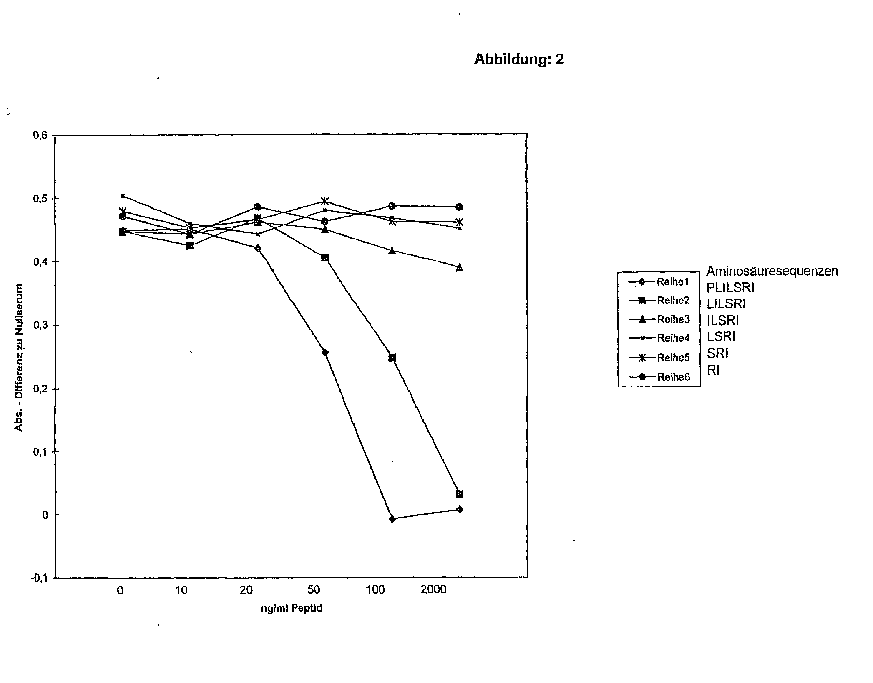

Wie im Beispiel 1 d) beschrieben, wurde proPSA aus PCa-Serum in mit Streptavidin vorbeschichteten Näpfen von Mikrotiterplatten gebunden. Anschließend wurden die anti-proPSA-Antikörper zusammen mit den unterschiedlich langen proPSA-Peptiden für 1 Stunde gemeinsam inkubiert. Die zugesetzten freien Peptide können in diesem Schritt mit proPSA aus dem PCa-Serum auf der Festphase um die Bindung an die Antikörper konkurrieren. Peptide, welche gut erkannt werden, führen zu einer Verdrängungsreaktion und damit letztendlich zu einer Signalerniedrigung, weil weniger spezifische Antilcörper und damit auch weniger Nachweisantikörper gebunden wurden. Wie aus Abbildung 2 hervorgeht, führen nur die Peptide [-6]proPSA und [-5] proPSA zu einer starken Verdrängungsreaktion. Das [-7]proPSA wurde in diesem Experiment nicht mitgeführt. In anderen Experimenten zeigte das [-7]proPSA-Peptid eine ähnlich gute Kompetition, wie das [-5] oder das [-6]proPSA-Peptid.

### Beispiel 4

### Nachweis von [-5, -6, -7]proPSA im ELISA

Der Nachweis von [-5, -6, -7] proPSA erfolgte in einem sogenannten Sandwich-ELISA mit dem proPSA-MAK als Fangantikörper und einem POD-marlcierten MAK gegen freies PSA als Nachweisreagenz. Der MAK 1.023.6 wurde über Standardverfahren biotinyliert und in einer Konzentration von 2,5 µg /ml eingesetzt. Dieser Test erfolgte analog zum Enzymun^{R}-Test auf freies PSA, mit dem wichtigen Unterschied, dass als Fangreagenz der erfindungsgemäße (biotinylierte) MAK 1.023.6 eingesetzt wurde

### Beispiel 5

### ROC-Analysen mit unterschiedlichen PSA-Fraktionen

In insgesamt 41 Proben mit einem Gesamt-PSA-Wert zwischen 4- 10 ng/ml, aber hinsichtlich BPH, bzw. PCa abgesicherter Diagnose, wurden die Parameter freies PSA und gesamt-PSA mit dem automatischen Immunanalyzer ELECSYS® (Roche Diagnostics), und proPSA entsprechend Beispiel 4 bestimmt. Die Quotienten aus freiem PSA zu gesamt-PSA, proPSA zu gesamt-PSA und freiem PSA zu proPSA wurden errechnet und ebenso wie die Einzelparameter einer ROC-Analyse unterworfen. Wie aus Abbildung 3 klar hervorgeht, ergibt sich für das Verhältnis von freiem PSA zu proPSA der größte AUC-Wert. Somit trägt dieser "Parameter" wesentlich besser zur Unterscheidung zwischen BPH und PCa bei als die beiden anderen Relativwerte oder die jeweiligen Einzelwerte.

## Patentansprüche

1. Antikörper mit Spezifität für alle der folgenden proPSA-Formen : [-5], [-6] und [-7] proPSA,
**dadurch gekennzeichnet, dass**
diese Antikörper mit [-4]-proPSA und kürzeren Formen des proPSA im Wesentlichen nicht reaktiv sind.

2. Antikörper nach Anspruch 1,
**dadurch gekennzeichnet, dass**
es sich bei diesen Antikörpern um monoklonale Antikörper handelt.

3. Antikörper nach Anspruch 1 oder 2,
**dadurch gekennzeichnet, dass**
es sich dabei um Antikörper handelt, welche im Wesentlichen die gleiche Bindeeigenschaften aufweisen, wie monoklonale Antikörper, welche von dem unter ACC 2456 bei der DSMZ hinterlegten Klon sezerniert werden.

4. Verfahren zur Herstellung von Antikörpern gegen alle der folgenden proPSA-Formen : [-5], [-6] und [-7] proPSA, unter Verwendung synthetischer trägergebundener Peptide, welche der proPeptid-Sequenz des [-5], [-6] und/oder [-7]proPSA entsprechen,
**dadurch gekennzeichnet, dass**
über das Screening-Verfahren Antikörper erhalten werden, welche im Wesentlichen nicht reaktiv sind mit [-4] proPSA oder kürzeren Formen des proPSA.

5. Methode zum Nachweis von allen der folgenden proPSA-Formen: [-5], [-6] und [-7] proPSA, in einer Probe
**dadurch gekennzeichnet, dass**
ein spezifischer Antikörper gemäß einem der Ansprüche 1 - 3 zum Nachweis eingesetzt wird.

6. Methode nach Anspruch 5,
**dadurch gekennzeichnet, dass**
als spezifischer Fangantikörper ein Antikörper gemäß Anspruch 1 - 3 eingesetzt wird.

7. Methode nach einem der Ansprüche 5 oder 6,
**dadurch gekennzeichnet, dass**
ein Fangantikörper mit Spezifität für alle der folgenden proPSA-Formen: [-5], [-6] und [-7] proPSA, sowie ein Nachweisantikörper mit Spezifität für freies PSA eingesetzt wird.

8. Methode nach einem der Ansprüche 5, 6, 7,
**dadurch gekennzeichnet, dass**
der spezifische Fangantikörper biotinyliert vorliegt.

9. Diagnostische Testzusammensetzung zum Nachweis von allen der folgenden proPSA-Formen : [-5], [-6] and [-7] proPSA, welche Antikörper mit Spezifität für alle der folgenden proPSA-Formen: [-5], [-6] und [-7] proPSA, welche im Wesentlichen nicht mit kürzeren proPSA Formen reaktiv sind, enthält

10. Verfahren zum Nachweis oder Ausschluß eines Prostata-Karzinoms
**dadurch gekennzeichnet, dass**
alle der folgenden proPSA-Formen: [-5], [-6] und [-7] proPSA in einer Probe bestimmt wird.

11. Verfahren zum Nachweis oder Ausschluß eines Prostata-Karzinoms
**dadurch gekennzeichnet, dass**
alle der folgenden proPSA-Formen: [-5], [-6] und [-7] proPSA in einer Probe bestimmt und zu anderen Kallikrein-Fraktionen oder zur Gesamtmenge eines oder mehrerer Kallikreine ins Verhältnis gesetzt wird

12. Verfahren nach Anspruch 11,
**dadurch gekennzeichnet, dass ,**
das Kallikrein PSA ist.

13. Verfahren zum Nachweis oder Ausschluß eines Prostata-Karzinoms
**dadurch gekennzeichnet, dass**
alle der folgenden proPSA-Formen: [-5], [-6] und [-7] proPSA und freies PSA in einer Probe bestimmt werden und
ein Quotient aus allen der folgenden proPSA-Formen: [-5], [-6], und [-7] proPSA und freiem PSA gebildet wird.

14. Verfahren zum Nachweis oder Ausschluß eines Prostata-Karzinoms,
**dadurch gekennzeichnet, dass**
alle der folgenden proPSA-Formen : [-5], [-6] und [-7] proPSA unter Verwendung der Antikörper gemäß Anspruch 1-3 aufgereinigt und jede dieser drei proPSA-Formen einzeln quantifiziert wird.

## Claims

1. Antibodies specific for all of the following proPSA forms: [-5], [-6] and [-7] proPSA,
**characterized in that**
these antibodies are essentially non-reactive with [-4] proPSA and shorter forms of proPSA.

2. Antibodies according to claim 1,
**characterized in that**
these antibodies are monoclonal antibodies.

3. Antibodies according to claim 1 or 2,
**characterized in that**
they are antibodies which have essentially the same binding properties as monoclonal antibodies which are secreted by the clone deposited under ACC 2456 at the DSMZ.

4. Process for producing antibodies against all of the following proPSA forms: [-5], [-6] and [-7] proPSA using synthetic carrier-bound peptides corresponding to the propeptide sequence of [-5], [-6] and/or [-7] proPSA,
**characterized in that**
antibodies are obtained by the screening process which are essentially not reactive with [-4] proPSA or shorter forms of proPSA.

5. Method for detecting all of the following proPSA forms: [-5], [-6] and [-7] proPSA in a sample,
**characterized in that**
a specific antibody according to one of the claims 1 - 3 is used for the detection.

6. Method according to claim 5,
**characterized in that**
an antibody according to claims 1- 3 is used as a specific capture antibody.

7. Method according to one of the claims 5 or 6,
**characterized in that** a capture antibody specific for all of the following proPSA forms: [-5], [-6] and [-7] proPSA as well as a detection antibody specific for free PSA are used.

8. Method according to one of the claims 5, 6 or 7,
**characterized in that**
the specific capture antibody is present in a biotinylated form.

9. Diagnostic test composition for detecting all of the following proPSA forms: [-5], [-6] and [-7] proPSA which contains antibodies specific for all of the following proPSA forms: [-5], [-6] and [-7] proPSA said antibodies being essentially non-reactive with shorter proPSA forms.

10. Method for detecting or excluding a prostate carcinoma,
**characterized in that**
all of the following proPSA forms: [-5], [-6] and [-7] proPSA are determined in a sample.

11. Method for detecting or excluding a prostate carcinoma,
**characterized in that**
all of the following proPSA forms: [-5], [-6] and [-7] proPSA are determined in a sample and expressed as a ratio to other kallikrein fractions or to the total amount of one or more kallikreins.

12. Method according to claim 11,
**characterized in that**
the kallikrein is PSA.

13. Method for detecting or excluding a prostate carcinoma,
**characterized in that**
all of the following proPSA forms: [-5], [-6] and [-7] proPSA and free PSA are determined in a sample and the ratio of all of the following proPSA forms: [-5], [-6] and [-7] proPSA to free PSA is calculated.

14. Method for detecting or excluding a prostate carcinoma,
**characterized in that**
all of the following proPSA forms: [-5], [-6] and [-7] proPSA are purified using the antibodies according to claims 1-3 and each of these three proPSA forms is quantified individually.

## Revendications

1. Anticorps manifestant une spécificité pour toutes les formes proPSA suivantes : [-5], [-6] et [-7] proPSA,
**caractérisés en ce que** ces anticorps ne manifestent pas de réactivité essentielle avec [-4] proPSA et avec des formes plus courtes du proPSA.

2. Anticorps selon la revendication 1,
**caractérisés en ce que**,
quant à ces anticorps, il s'agit d'anticorps monoclonaux.

3. Anticorps selon la revendication 1 ou 2,
**caractérisés en ce que**,
il s'agit en l'occurrence d'anticorps qui présentent essentiellement les mêmes propriétés de liaison que celles des anticorps monoclonaux qui sont sécrétés à partir du clone déposé à la DSMZ sous le numéro matricule ACC 2456.

4. Procédé pour la préparation d'anticorps dirigés contre toutes les formes proPSA suivantes : [-5], [-6] et [-7] proPSA, en utilisant des peptides synthétiques liés à un support, qui correspondent à la séquence propeptidique des [-5], [-6] et/ou [-7] proPSA,
**caractérisé en ce que**
on obtient, via le procédé de criblage, des anticorps qui ne manifestent pas de réactivité essentielle avec [-4] proPSA ou avec des formes plus courtes du proPSA.

5. Procédé pour le décèlement de toutes les formes proPSA suivantes : [-5], [-6] et [-7] proPSA dans un échantillon,
**caractérisé en ce que**
on met en oeuvre, pour le décèlement, un anticorps spécifique selon l'une quelconque des revendications 1 à 3.

6. Procédé selon la revendication 5,
**caractérisé en ce que**
on met en oeuvre, à titre d'anticorps de capture spécifique, un anticorps selon l'une quelconque des revendications 1 à 3.

7. Procédé selon l'une quelconque des revendications 5 ou 6,
**caractérisé en ce que**
on met en oeuvre un anticorps de capture manifestant une spécificité pour toutes les formes proPSA suivantes : [-5], [-6] et [-7] proPSA, ainsi qu'un anticorps de décèlement manifestant une spécificité pour le PSA libre.

8. Procédé selon l'une quelconque des revendications 5, 6, 7,
**caractérisé en ce que**
l'anticorps de capture spécifique est présent sous forme biotinylée.

9. Composition d'essai diagnostique pour le décèlement de toutes les formes proPSA suivantes : [-5], [-6] et [-7] proPSA, qui contient des anticorps manifestant une spécificité pour toutes les formes proPSA suivantes : [-5], [-6] et [-7] proPSA, qui ne manifestent pas de réactivité essentielle avec des formes plus courtes du proPSA.

10. Procédé pour le décèlement ou l'exclusion d'un carcinome de la prostate,
**caractérisé en ce que**
on détermine toutes les formes proPSA suivantes : [-5], [-6] et [-7] proPSA dans un échantillon.

11. Procédé pour le décèlement ou l'exclusion d'un carcinome de la prostate,
**caractérisé en ce que**
on détermine toutes les formes proPSA suivantes : [-5], [-6] et [-7] proPSA dans un échantillon et on les met en rapport avec d'autres fractions de kallicréines ou avec la quantité totale d'une ou de plusieurs kallicréines.

12. Procédé selon la revendication 11,
**caractérisé en ce que**
la kallicréine est le PSA.

13. Procédé pour le décèlement ou l'exclusion d'un carcinome de la prostate,
**caractérisé en ce que**
on détermine toutes les formes proPSA suivantes : [-5], [-6] et [-7] proPSA et le PSA libre dans un échantillon et on calcule un quotient à partir de toutes les formes proPSA suivantes : [-5], [-6] et [-7] proPSA et du PSA libre.

14. Procédé pour le décèlement ou l'exclusion d'un carcinome de la prostate,
**caractérisé en ce que**
on purifie toutes les formes proPSA suivantes : [-5], [-6] et [-7] proPSA en utilisant les anticorps selon les revendications 1 - 3 et on procède à la quantification individuelle de chacune de ces trois formes de proPSA.
